# EUROPEAN PATENT APPLICATION

(11) **EP 4 790 127 A1**
(43) Date of publication of application: **12.08.2026**
(21) Application number: 25227784.3
(22) Date of filing: 30.12.2025
(51) Int. Cl.: C12M 1/00, C12M 1/107, C02F 11/02

(54) **SYSTEM AND METHOD FOR PROCESSING EFFLUENTS AND USE IN THE PRODUCTION OF MICROALGAE**

(30) Priority: 30.12.2024 PT 2024119959
(71) Applicant: Oprimee - Innovation Design Engineering Solutions, Lda, 3405-155 Oliveira do Hospital (PT)
(72) Inventor: DOS SANTOS ALMEIDA NUNES, JOÃO MIGUEL, 3405-155 OLIVEIRA DO HOSPITAL (PT)
(74) Representative: Patentree

(57) **Abstract**

A system and method are provided for processing an industrial effluent and cultivating microalgae. The system comprises a primary tank, a screening unit with a motor-driven cleaning mechanism, a flocculation tank supplied with a chemical flocculant, a sand filter, an acidification unit with pH control, a solid-liquid separation unit, an anaerobic digestion unit, and a microalga cultivation unit. The effluent is screened, flocculated, filtered, acidified to a pH from 4.2 to 5.2, preferably 4.45 to 4.75, and subjected to solid-liquid separation to remove precipitated proteins and destabilised colloidal material, thereby producing a clarified effluent. The clarified effluent is anaerobically digested at a defined organic loading rate to generate biogas and a liquid digestate. At least a portion of the digestate and/or carbon dioxide derived from the biogas is used for microalga cultivation. The system enables stable anaerobic digestion without membrane-based pretreatment and provides a conditioned stream suitable for downstream microalga growth.

## Description

### TECHNICAL FIELD

The present disclosure relates to the field of industrial effluent processing, particularly effluents generated by the dairy industry in the production of cheese. In particular, it relates to a system and method that not only treats effluents generated by the dairy industry but also enables the simultaneous cultivation of microalgae using byproducts from the effluent treatment process.

### BACKGROUND

The dairy industry, particularly the cheese manufacturing sector, is a significant contributor to global food production, with cheese being a widely consumed product. Cheese is produced from milk, which varies in composition depending on the animal source, including cows, sheep, goats, and buffalo. The milk's composition can also be influenced by factors such as the animal's breed, diet, and stage of lactation. The main components of milk include fats, proteins, lactose, minerals, and vitamins, and during cheese production, milk is separated into curd (rich in fats and proteins) and whey (rich in proteins, lactose, vitamins, and minerals).

The whey produced during cheese manufacturing has a wide range of applications in food and non-food products due to its nutritional value and functional properties. These include its use in dairy products such as yogurt, ice cream, and chocolate beverages, as well as in baked goods, meat and fish products, pharmaceuticals, and dietary supplements.

Despite its valuable properties, whey, along with other by-products such as second whey from curd cheese production and wash waters, also constitutes a significant environmental challenge. It is estimated that for every litter of milk processed, between 1 and 6 litters of liquid effluents are generated. These effluents can contain high levels of organic matter, which, if discharged untreated, lead to severe environmental consequences such as oxygen depletion, eutrophication, contamination of water bodies, and potential threats to biodiversity. Historically, untreated effluents were often discharged directly into watercourses or land, resulting in detrimental impacts on aquatic and terrestrial ecosystems.

Nowadays, the dairy industry faces increasing pressure to adopt sustainable practices to manage these waste streams effectively. Traditional wastewater treatment methods, including biological processes (both anaerobic and aerobic digestion), have been employed but are often inadequate in fully addressing the complexities of cheese production effluents. These biological treatments can be limited by slow retention times, difficulty in breaking down lactose and proteins, and the production of excess sludge and unpleasant odours. Furthermore, such treatments may struggle with the high fat content and suspended solids found in dairy effluents, necessitating additional steps such as flotation or coagulation.

Several conventional treatment processes have been proposed, with a focus on physical-chemical, biological, and combined methods. Physical-chemical treatments include coagulation-flocculation, precipitation (both acid and basic), electrocoagulation, and advanced methods like osmotic filtration or reverse osmosis. These methods aim to remove suspended solids, reduce organic load, and minimize the environmental impact of wastewater discharge. Biological treatments, on the other hand, rely on the activity of microorganisms to degrade organic matter, with the potential for either aerobic or anaerobic processes depending on the specific characteristics of the wastewater.

However, there remains an ongoing need for innovative and cost-effective methods that can efficiently treat the effluents from cheese production, particularly given the complexity of the wastewater's composition. Current methods may not always meet the stringent environmental regulations, and new approaches that can improve treatment efficiency while minimizing environmental impact are crucial.

In this context, this invention seeks to provide an innovative method for treating dairy effluents, specifically those generated in cheese production, by addressing the limitations of conventional methods and offering a more sustainable, economically viable solution.

Traditional effluent processing systems and methods are often energy-intensive, and the byproducts generated are typically not reused. Therefore, there is a need for a solution that not only effectively treats these effluents but also enables the recovery and utilization of valuable byproducts, promoting sustainable practices and supporting a circular economy. This solution would contribute to the production of high-value products, further enhancing the environmental and economic benefits of effluent management.

Various approaches have been proposed to selectively enrich or favour microbial populations that promote hydrogen production or acidogenic fermentation. One known method, described for example in US2006289355A1, involves treating a biomass containing hydrogen-producing bacteria and competing bacteria with a chemical agent for a defined period of time. The chemical treatment is selected such that substantially all competing bacteria are killed, inhibited, or injured, while hydrogen-producing bacteria remain viable or form spores capable of surviving the treatment. The resulting enriched biomass can then be mixed with a non-sterile organic substrate and digested under conditions that allow the hydrogen-producing bacteria to dominate and generate hydrogen.

Another approach to controlling microbial activity in waste conversion processes is described in US2021309549A1. This document discloses methods for converting organic waste streams by fermentation using an inoculum subjected to arrested methanogenesis. The inoculum comprises an anaerobic consortium isolated from sources such as cheese, yogurt, saline soil, kefir, or probiotic formulations, and is pretreated to transform the anaerobic consortium into an acidogenic consortium. By inhibiting methanogenic activity, the process promotes the production of organic acids or other intermediate products rather than methane.

In addition to biological enrichment and fermentation strategies, integrated process schemes combining biological and thermochemical conversion have also been proposed. For example, US2020156973A1 describes the integration of a gas fermentation process with a gasification process. In this system, one or more effluents generated downstream of the gas fermentation process, including biogas, tail gas, microbial biomass, wastewater, or liquid by-products, are recycled to a gasification unit. The gasification process generates syngas, which may be reused in the fermentation process, thereby improving overall resource efficiency and process integration.

These facts are disclosed in order to illustrate the technical problem addressed by the present disclosure.

### GENERAL DESCRIPTION

The present disclosure relates to a system and a method for processing industrial effluents generated by the dairy industry, in particular in the production of cheese, and their use in the production of microalgae.

An aspect of the present disclosure relates to a system and method for processing an industrial effluent generated by the dairy industry, in particular in the production of cheese, and their use in the production of microalgae, comprising: a primary tank for the initial collection of effluent; sieve tank for solid-liquid separation; a chemical flocculation unit with air injection to remove suspended particles; gravity sand filter for fine particle filtration; an anaerobic digestion reactor for the degradation of organic content; system for recovering and utilizing biogas; microalgae cultivation unit for the production of biostimulants.

The present disclosure integrates a sequence of physical-chemical and biological treatment steps that not only processes the effluent effectively, removing organic contaminants efficiently, but also allow for the valorisation of the by-products generated, such as biogas, creating a closed-loop system that reduces waste and improves resource recovery. Surprisingly, the present disclosure exhibits improved energy efficiency by utilizing biogas as a power source for at least one stage of the process. Additionally, biogas serves also as a source of carbon dioxide for the growth of microalga, which can be applied as biostimulants, thereby promoting a circular economy within the system by reusing energy and resources, making the process more self-sustainable and energy-efficient. The system contributes to a circular economy by using effluent byproducts to support microalgae production, promoting both environmental sustainability and economic efficiency.

In an embodiment, the anaerobic digestion reactor may generate a biogas.

In an embodiment, the biogas may be the energy source to power at least one next stage of the process to improve the system energy efficiency.

In an embodiment, the biogas may act as a source of CO₂ to grow microalgae, promoting a circular economy within the system.

Another aspect of the present disclosure relates to a method for processing an effluent generated in the production of cheese and their use in the production of microalgae, comprising the following steps: providing the effluent; transferring the effluent to a sieve tank parallel to the primary tank, where solid-liquid separation takes place; mechanical agitation of the effluent in the sieve tank to separate the solid and liquid phases; addition of a flocculating agent to the liquid phase in the sieve tank to conduct chemical flocculation; air supply through a blower to increase the removal efficiency of suspended particles, facilitating the agglomeration of solids; mechanical agitation of the effluent in the sieve tank in order to split the solid and liquid phases; addition of a flocculant agent to the liquid phase in the sieve tank to perform chemical flocculation; air supply through a blower to increase the efficiency of suspended particle removal, thereby simplifying the agglomeration of solids; passage of the filtered effluent through a gravity sand filter; anaerobic digestion of the filtered effluent to degrade the organic content in an oxygen-free environment; using the biogas generated in the anaerobic digestion process as an energy source, improving the reaction kinetics; cultivation of microalgae using CO₂ as a catalyst for the production of biostimulants.

Surprisingly, it has been found that protein-rich industrial effluents, in particular dairy effluents, can be conditioned in a technically simple and robust manner such that stable anaerobic digestion is achieved without membrane-based pretreatment, while simultaneously producing a stream suitable for downstream microalga cultivation.

In known effluent treatment systems, residual proteins and proteinaceous colloids typically cause operational instability during anaerobic digestion and impair subsequent microalga growth, thereby requiring complex pretreatment steps or membrane filtration. The technical problem addressed by the present invention is therefore to provide a system and method for processing such effluents that enable stable anaerobic digestion and effective integration with microalga cultivation using non-sterile, membrane-free process steps.

This problem is solved by to a system and method for processing an industrial effluent and cultivating microalgae. The system comprises a primary tank, a screening unit with a motor-driven cleaning mechanism, a flocculation tank supplied with a chemical flocculant, a sand filter, an acidification unit with pH control, a solid-liquid separation unit, an anaerobic digestion unit, and a microalga cultivation unit. The effluent is screened, flocculated, filtered, acidified to a pH from 4.2 to 5.2, preferably 4.4 to 4.75, and subjected to solid-liquid separation to remove precipitated proteins and destabilised colloidal material, thereby producing a clarified effluent. The clarified effluent is anaerobically digested at a defined organic loading rate to generate biogas and a liquid digestate. At least a portion of the digestate and/or carbon dioxide derived from the biogas is used for microalga cultivation. The system enables stable anaerobic digestion without membrane-based pretreatment and provides a conditioned stream suitable for downstream microalga growth.

An aspect of the present disclosure relates to a system for processing an effluent and produce a microalga, comprising a primary tank; a second tank connected to the primary tank configured for flocculation; a unit formed by a motor connected to paddles placed above a sieve positioned at the upper edge of the second tank, wherein the motor drives the paddles to clean the sieve; a container for a chemical flocculator linked to a third tank, wherein the third tank has a blower configured to supply air in the base of the third tank and a particle suspension in the upper edge; a sand filter; an unit configured to perform anaerobic digestion by a microorganism, wherein the anaerobic digestion results in the production of compost; a microalga cultivation unit.

An effluent refers to a liquid waste stream generated by an industrial process, comprising dissolved and suspended organic matter, nutrients, and water. In particular, an effluent generated by the dairy industry refers to a liquid waste stream originating from milk processing, cheese production, or related dairy operations and typically containing proteins, fats, lactose, and colloidal material.

A primary tank designates a vessel configured to receive and temporarily store the effluent prior to further processing. A screening unit designates a mechanical separation device comprising a sieve configured to retain solid material while allowing a liquid fraction to pass through. A motor-driven cleaning mechanism refers to a mechanical arrangement comprising a motor and moving elements configured to remove retained solids from the sieve during operation.

A flocculation tank designates a vessel configured to promote aggregation of suspended particles or colloids by addition of a chemical flocculant. A chemical flocculant refers to a chemical agent added to the effluent to induce aggregation or destabilisation of suspended or colloidal material, and may comprise an acid.

A sand filter designates a filtration unit containing granular material configured to remove remaining suspended solids from the effluent by physical filtration.

An acidification unit designates a processing unit comprising an acid dosing device and a pH control unit, the pH control unit comprising at least one pH sensor and a feedback control loop configured to regulate acid addition. Acidification refers to adjustment of the pH of the effluent by addition of an acid. A pH range from 4.45 to 4.75 refers to the pH of the effluent measured at 25 °C using a calibrated pH electrode immersed in the liquid phase.

A solid-liquid separation unit designates a unit configured to separate precipitated or aggregated solid material from a liquid phase, and may comprise a gravitational settler, a dissolved air flotation unit, or a combination thereof.

A clarified effluent refers to a liquid stream obtained after solid-liquid separation and having a reduced content of suspended solids, proteins, and proteinaceous colloids relative to the untreated effluent.

An anaerobic digestion unit designates a reactor configured to biologically convert dissolved organic matter in the absence of oxygen by anaerobic microorganisms. Anaerobic microorganisms refer to bacteria and/or archaea capable of metabolising organic compounds under anaerobic conditions. Anaerobic digestion refers to the biological conversion of organic matter into biogas and a liquid digestate.

Organic loading rate refers to the mass of chemical oxygen demand (COD) supplied to the anaerobic digestion unit per unit reactor volume and per day, expressed in kilograms of COD per cubic metre per day. COD refers to chemical oxygen demand determined according to standard analytical methods.

Biogas refers to a gaseous product generated by anaerobic digestion and comprising methane and/or carbon dioxide. Carbon dioxide derived from biogas refers to carbon dioxide separated from the biogas stream or present in the biogas and made available for downstream use.

A liquid digestate refers to the liquid phase discharged from the anaerobic digestion unit after biological conversion of organic matter and containing water, dissolved nutrients, and residual organic compounds.

A microalga cultivation unit designates a reactor or cultivation system configured to support the growth of microalgae in an aqueous medium under illumination. Microalgae refer to unicellular or simple multicellular photosynthetic microorganisms capable of growth in aqueous environments. Cultivation of microalgae refers to the growth of microalgae using nutrients and, optionally, carbon dioxide supplied from the liquid digestate and/or biogas derived from anaerobic digestion.

Stable anaerobic digestion refers to continuous operation of the anaerobic digestion unit without process failure, excessive foaming, or biomass washout, under the defined organic loading rate.

An aspect of the disclosure is related with a system for processing an effluent and cultivating a microalga, comprising: a primary tank configured to receive the effluent; a screening unit fluidly connected downstream of the primary tank, the screening unit comprising a sieve and a motor-driven cleaning mechanism configured to remove solid matter retained on the sieve; a flocculation tank arranged downstream of the screening unit with a container for a flocculant agent fluidly connected to the flocculation tank; a sand filter arranged downstream of the flocculation tank; an acidification unit arranged downstream of the sand filter to adjust pH to 4.2-5.2, preferably 4.45-4.75, prior to solid-liquid separation; a solid-liquid separation unit arranged downstream of the acidification unit and configured to remove precipitated proteins and destabilised colloidal material, thereby producing a clarified effluent; an anaerobic digestion unit fluidly connected downstream of the solid-liquid separation unit and configured to receive the clarified effluent at an organic loading rate from 2 to 6 kg COD per m³ per day and to convert dissolved organic matter into biogas and a liquid digestate; and a microalga cultivation unit fluidly configured to receive at least a portion of the liquid digestate and/or carbon dioxide derived from the biogas connected to receive at least a portion of the liquid digestate and/or gaseous products, including and/or carbon dioxide derived from the biogas, of the anaerobic digestion unit, wherein the acidification unit is configured to adjust the effluent to a pH from 4.2 to 5.2 prior to the solid-liquid separation unit.

In an embodiment, the effluent may be an effluent generated by the dairy industry, preferably in production of cheese.

In an embodiment, the sieve may be made of ferrous or non-ferrous metals, plastic, ceramics, or composite materials.

In an embodiment, the sieve may be made of stainless steel, carbon steel, brass, aluminum, copper, bronze, nickel or titanium, among others.

In an embodiment, the sieve mesh size may be 10-1000 µm; preferably 20-500 µm; preferably 50-400 µm.

In an embodiment, the chemical flocculator may be an acid; preferably a strong inorganic acid.

In an embodiment, the flocculant agent may be selected from a list consisting of: hydrochloric acid (HCl), sulfuric acid (H₂SO₄), and nitric acid (HNO₃); preferably hydrochloric acid (HCl).

In an embodiment, the microorganism anaerobic digestion may generate a biogas; preferably methane (CH₄) and/or carbon dioxide (CO₂); more preferably carbon dioxide (CO₂).

In an embodiment, the biogas may be the energy source to power at least the next stage of the process, namely the microalga cultivation.

In an embodiment, the compost may comprise at least organic matter, nutrients, assimilable nitrogen, phosphorus and water suitable for microalga cultivation.

In an embodiment, the biogas generated in the anaerobic digestion unit of the system is used as an internal energy source to power at least one unit of the system.

In an embodiment, the microalga may be selected from a list consisting of: *Chlorella, Spirulina, Dunaliella, Scenedesmus, Nannochloropsis, Tetraselmis, Isochrysis, Phaeodactylum, Chlamydomonas, Arthrospiro, Navicula, Botryococcus, Pseudokirchneriella, Cyclotella,* or *Crypthecodinium,* among others.

In an embodiment, the system operates without reverse osmosis, nanofiltration, ultrafiltration or microfiltration units.

Another aspect of the present disclosure relates to a method for processing an effluent and produce microalgae, comprising the following steps: providing the effluent; introducing the effluent into the primary tank; loading the effluent into the sieve tank; separating the effluent using the sieve into a liquid and a solid phase; cleaning the solid phase from the sieve using the paddles controlled by the motor; transferring the liquid phase to the third tank; adding the chemical flocculator of the container to the liquid phase in the third tank to remove particles in suspension; filtering the liquid phase, without suspended particles, in the sand filter; transferring the filtered liquid phase to the unit configured to perform anaerobic digestion by a microorganism; digesting the organic matter of the liquid phase by the microorganisms resulting in a compost and CO₂; using the compost, rich in nutrients and the CO₂, in a microalga cultivation unit.

Another aspect of the present disclosure relates to the use of the microalga obtained by the method of the present disclosure as a biostimulant.

Another aspect of the present disclosure relates to a method for processing an effluent and produce microalgae, comprising the following steps: providing an effluent; introducing the effluent into a primary tank; screening the effluent using a sieve to separate a solid fraction from a liquid fraction; removing retained solids from the sieve using a motor-driven cleaning mechanism; subjecting the liquid fraction to flocculation by adding a chemical flocculant; filtering the flocculated liquid fraction using a sand filter; acidifying the filtered liquid fraction to a pH from 4.2 to 5.2, preferably 4.45 to 4.75 prior to solid-liquid separation; solid-liquid separating unit configured to remove precipitated proteins and destabilised colloids to produce a clarified effluent; anaerobic digestion unit operated at 2-6 kg COD·m⁻³·day⁻¹ producing biogas and liquid digestate; and microalga cultivation unit receiving at least a portion of digestate and/or CO₂ derived from biogas.

### BRIEF DESCRIPTION OF THE DRAWINGS

The following figures provide preferred embodiments for illustrating the disclosure and should not be seen as limiting the scope of invention.

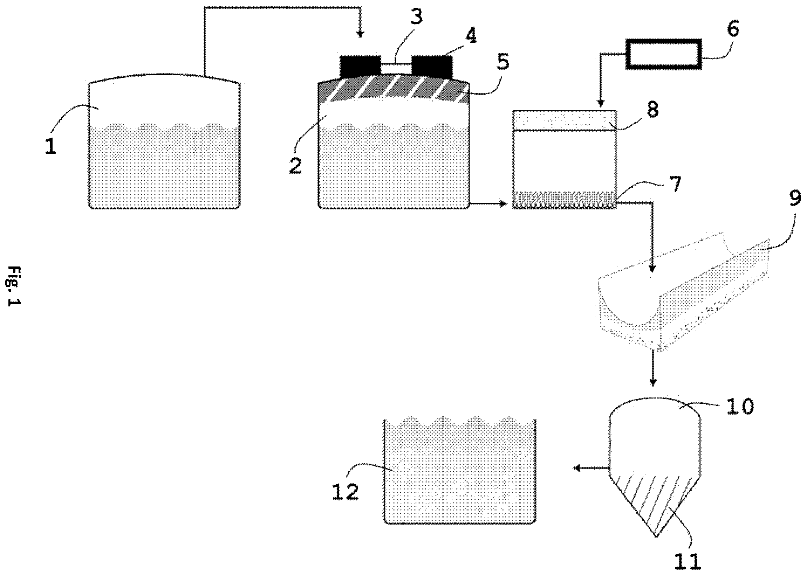

**Figure 1****:** Schematic representation of the system and method of processing the effluent from the dairy industry, showing the primary tank (1) connected in parallel to the screen tank (2). The system includes several components, such as the chemical flocculation unit with flocculant (6), the blower (7), and the gravity sand filter (9). The anaerobic digestion reactor (10) and the microalgae cultivation unit (12) are also depicted, highlighting the effluent flow and the key processes for treatment and resource recovery.

### DETAILED DESCRIPTION

A system and method are provided for processing an industrial effluent and cultivating microalgae. The system comprises a primary tank, a screening unit with a motor-driven cleaning mechanism, a flocculation tank supplied with a chemical flocculant, a sand filter, an acidification unit with pH control, a solid-liquid separation unit, an anaerobic digestion unit, and a microalga cultivation unit. The effluent is screened, flocculated, filtered, acidified to a pH from 4.2 to 5.2, preferably 4.45 to 4.75, and subjected to solid-liquid separation to remove precipitated proteins and destabilised colloidal material, thereby producing a clarified effluent. The clarified effluent is anaerobically digested at a defined organic loading rate to generate biogas and a liquid digestate. At least a portion of the digestate and/or carbon dioxide derived from the biogas is used for microalga cultivation. The system enables stable anaerobic digestion without membrane-based pretreatment and provides a conditioned stream suitable for downstream microalga growth.

The present disclosure relates to a system and a method for processing industrial effluents generated by the dairy industry, particularly in the production of cheese and the use of byproducts of the effluent processing in the production of microalgae. The present disclosure integrates several physical-chemical and biological steps to effectively remove organic contaminants and reuse by-products, such as nutrients, CO₂, and organic matter, to support the growth of microalgae. These microalgae can then be harvested and processed into biostimulants. By creating a circular process, the system promotes resource recovery, energy efficiency, and ecological sustainability.

The present disclosure relates to a system and method for the processing of industrial effluents generated by the dairy industry, in particular in the cheese production, and their use in the production of microalgae.

The present disclosure not only aims to significantly reduce the environmental load of the dairy industry's effluents, but also offers additional and valuable applications for the by-products generated during treatment, such as the production of microalgae, known as biostimulants for agriculture, creating a sustainable cycle.

In an embodiment, briefly, the process involves loading effluent into a primary tank, which is connected in parallel to a sieve tank. In the sieve tank, the physical-chemical separation of the solid and liquid phases takes place, with the liquid phase going through a chemical flocculation treatment, where a flocculant is added to remove suspended particles. The efficiency of the process is increased by air introduction through a blower. Afterwards, the effluent is filtered through a sand filter by gravity and then subjected to an anaerobic digestion process, where the degradation of organic matter takes place in an oxygen-free environment. The biogas generated during digestion is used as an energy source for the system, while the CO₂ present in the biogas favors the growth of microalgae. These microalgae can be used to produce biostimulants, which can be used in agriculture.

### EXAMPLE 1

Figure 1 represents an embodiment of the present disclosure and depicts the innovative and sustainable system described in the present disclosure for the processing of effluents generated by the dairy industry, in particular in the cheese production process. The system includes several stages of physical-chemical and biological treatment, aimed at the effective removal of organic contaminants and the reuse of by-products, helping to minimise environmental impact.

In an embodiment, the method involves loading the effluent into a primary tank (1) which is connected in parallel to another tank, the sieve tank (2). The sieve tank is equipped with a motor (3) which drives paddles (4) responsible for cleaning the sieve (5), allowing the solid and liquid phases to be effectively separated. At this stage, the organic load present in the effluent is separated, with the liquid phase being sent to subsequent treatment stages.

In an embodiment, the liquid water from the sieve tank (2) then goes through a chemical flocculation process, where a chemical flocculator (6) is added. This process is reinforced by a blower (7), a device that supplies air and increases the efficiency of removing suspended particles (8), facilitating the agglomeration and solid precipitation.

In an embodiment, after flocculation, the effluent is filtered using a gravity sand filter (9). After this filtration, an anaerobic digestion process takes place (10), which aims to degrade organic matter through the action of microorganisms in an environment without the presence of oxygen. This treatment is complemented by the use of biogas from the digestion process itself, which acts as a source of energy for the system. The presence of CO₂ in the biogas increases the conductivity of the medium, accelerating the reaction kinetics and improving the efficiency of the anaerobic digestion process.

In an embodiment, the result of the anaerobic digestion process is a compost (11), which contains nutrients and substances that can be utilised in various ways. This compost (11), combined with the CO₂ from the biogas, which acts as a catalyst for the growth of microalgae, can be used in their production (12). The microalgae generated can be used to produce biostimulants, substances that have various applications, including as fertilisers in agriculture, promoting increased productivity and improved soil quality.

The present disclosure is an integrated and sustainable approach, which not only treats effluents efficiently, but also exploits the by-products generated, creating a virtuous cycle that reduces dependence on external chemicals and promotes the reuse of resources.

In an embodiment, the treatment process comprises a controlled flocculation step based on acidification, designed to promote the precipitation of residual casein and associated protein colloids present in effluents from the cheese industry, in particular cheese whey ("sweet whey") and scotta (sorelho) generated during ricotta or cream cheese production. This flocculation step constitutes a key physicochemical stage upstream of biological treatment, contributing to the reduction of suspended solids, clarification of the effluent, and stabilization of downstream anaerobic digestion.

Cheese whey derived from enzymatic, or rennet coagulation processes typically exhibits an initial pH ranging from 5.9 to 6.6, with a representative reference value of about 6.2. Acid whey, when present, generally has a lower initial pH, typically between 4.3 and 4.6, and may already lie close to the precipitation range. Scotta, obtained after heating whey during ricotta or cream cheese production, typically exhibits an initial pH in the range of 5.7 to 6.1, depending on the degree of prior acidification, thermal history, and storage conditions. These effluents are characterized by a high fraction of dissolved organic matter, mainly lactose, residual proteins including casein fractions, colloidal material, variable suspended solids associated with curd fines, fats, and cleaning operations, as well as significant concentrations of nutrients such as nitrogen, phosphorus, and potassium.

The flocculation process is based on acidifying the effluent to a pH close to the isoelectric point of casein, which is approximately pH 4.6. At this pH, the solubility of casein reaches a minimum, resulting in colloidal destabilization and aggregation of residual casein and associated proteinaceous and colloidal matter into flocs that can be physically separated. In preferred embodiments, the target pH for precipitation is set at a range from 4.45 to 4.75. This range ensures robust precipitation while accommodating variability in effluent composition. For optimization purposes, additional operating pH are possible, such as pH values around 4.2 or in the range of 5.0 to 5.2, particularly to account for matrix variability between whey and scotta, without departing from the scope of the invention.

In an embodiment, acidification is preferably carried out using strong inorganic acids, selected from hydrochloric acid, sulfuric acid, or combinations thereof. Hydrochloric acid is particularly preferred due to its high effectiveness in pH reduction and ease of fine control, while sulfuric acid may be used as an alternative when economic considerations justify its selection, provided that sulfate discharge limits and corrosion aspects are taken into account. For operational safety, homogeneous mixing, and prevention of localized over-acidification or gel formation, the acid is preferably dosed in diluted form, typically at concentrations from 5% to 10% by mass, and introduced into a rapid mixing zone.

The acid consumption required to reach the target pH is primarily governed by the alkalinity and buffering capacity of the effluent, which can vary widely depending on process conditions and mineralization. For preliminary design and start-up operation, representative alkalinity classes may be considered, such as low alkalinity around 200 mg/L as CaCO₃, medium alkalinity around 600 mg/L as CaCO₃, and high alkalinity around 1200 mg/L as CaCO₃. A practical approximation for acid demand estimation is based on the conversion of alkalinity to hydrogen ion equivalents, where the required moles of H⁺ per cubic meter are approximately equal to the alkalinity expressed in mg/L as CaCO₃ divided by 50. To account for additional buffering effects from proteins and dissolved salts typical of dairy matrices, a safety margin of approximately 20% is preferably applied.

Using these assumptions, the estimated consumption of hydrochloric acid at 33% concentration typically ranges from about 0.5 L/m³ for low alkalinity effluents up to about 3.0 L/m³ for highly alkaline effluents. Corresponding values for sulfuric acid at 98% concentration typically range from about 0.13 L/m³ to about 0.80 L/m³. For conservative design, a base value of approximately 1.5 L/m³ of HCl 33% may be adopted for sweet whey, while a higher base value of approximately 3.0 L/m³ of HCl 33% may be adopted for scotta, reflecting its generally higher mineralization and variability. Final dosing is preferably refined by titration curves or jar-test experiments conducted separately for each effluent stream.

The flocculation process is preferably carried out following a sequence of hydrodynamic conditions derived from standard jar-test protocols widely used in coagulation-flocculation optimization. After acid addition, a rapid mixing phase is applied for 30 to 60 seconds at high shear, typically corresponding to about 200 rpm in laboratory-scale testing, to ensure rapid dispersion of the acid and stabilization of the pH throughout the effluent volume. This is followed by a slow mixing phase lasting 15 to 25 minutes at low shear, typically corresponding from 25 to 45 rpm, to promote controlled growth of flocs without fragmentation. Subsequently, a clarification or settling phase is applied, typically lasting from 30 to 60 minutes, allowing gravitational separation of the formed flocs.

The process is preferably operated at temperatures from 20°C to 30°C, which provide favorable kinetics for flocculation and sedimentation. At lower temperatures, for example around 15°C, floc formation and settling rates may decrease, and seasonal verification through additional jar-tests may be performed to adjust operating conditions accordingly.

The efficiency of flocculation is enhanced when the effluent contains a sufficient concentration of suspended solids to promote particle collisions and nucleation. Favorable operating ranges typically correspond to suspended solids concentrations from 200 to 2000 mg/L. When the effluent is very clear but exhibits high dissolved chemical oxygen demand, flocculation efficiency may decrease, and optional measures such as recirculation of settled sludge, seeding, or the addition of low concentrations of polymeric flocculation aids, typically in the range of 0.5 to 8 mg/L, may be employed following jar-test validation.

The acidification and flocculation process results in the formation of a protein-rich sludge, predominantly composed of precipitated casein and associated colloidal material. For design purposes, the production of raw sludge typically ranges from 0.5% to 2% by volume of the treated flow for sweet whey and from 1% to 3% by volume for scotta. The dry solids content of the raw sludge typically ranges from 0.5% to 2% by mass. Following simple thickening operations, such as gravitational thickening or dissolved air flotation with thickening, solids concentrations in the range of 3% to 8% by mass can be achieved.

Solid-liquid separation following flocculation may be carried out by gravitational settling or, preferably in dairy matrices rich in proteins and fats, by dissolved air flotation. Flotation is particularly advantageous when flocs exhibit low apparent density and a tendency to float, providing faster clarification and higher operational robustness. Settling may be selected when flocs are denser and fat content is low, offering a simpler solution with reduced capital and operating costs.

The flocculation stage is preferably integrated with an automated control and instrumentation system. Control is centered on a closed-loop pH regulation, comprising an in-line pH probe, a dosing system operating proportionally to effluent flow, and a fine adjustment loop to maintain the pH setpoint at 4.60. Safety interlocks are preferably implemented, including alarms and automatic dosing cut-off when pH drops below 4.3, and deviation alarms when pH exceeds 4.9, to prevent over-acidification and process instability. Additional monitoring parameters may include flow rate, temperature, conductivity, and preferably turbidity or suspended solids concentration at the outlet of the clarification stage.

In an embodiment, the clarified effluent obtained after flocculation is directed to an anaerobic digestion unit. The digestion is preferably operated under mesophilic conditions at temperatures ranging from 35 °C to 38 °C, providing high robustness and stability, although thermophilic operation from 50 °C to 55 °C may also be employed. Typical hydraulic retention times range from 15 to 30 days, with organic loading rates from 2 to 6 kg COD per cubic meter per day. The digestion process converts dissolved organic matter into biogas, typically comprising from 55 to 65% methane and from 35 to 45% carbon dioxide, with minor amounts of hydrogen sulfide and water vapor. Potential inhibitors such as elevated salinity, fats, long-chain fatty acids, or nutrient imbalances may be managed by dilution, process control, or staged digestion.

Downstream of anaerobic digestion, the liquid digestate may be further valorized through microalgae cultivation. In this embodiment, the digestate, optionally filtered to remove residual fine solids, is used as a nutrient-rich growth medium. Cultivation is preferably carried out under controlled conditions, with photoperiods of 12 to 16 hours of light per day, light intensities in the range of 100 to 300 µmol photons per square meter per second, temperatures from 20°C to 30 °C, and operational pH values from 7.0 to 8.5. Carbon dioxide, derived at least in part from the biogas produced during anaerobic digestion, may be injected at concentrations from 2 to 10% by volume in the gas phase to control pH and support algal growth.

Suitable microalgae species include, without limitation, species of the genera Chlorella, Scenedesmus, Spirulina, and Nannochloropsis. The digestate provides assimilable nitrogen, predominantly in ammoniacal form, and phosphorus in the form of orthophosphate, as well as potassium. Excess salinity or ammonia concentrations may be managed by dilution. Cultivation is preferably carried out using the liquid phase, while coarse solids are excluded.

The integrated process enables a positive energy balance. Typical energy consumption values range from 0.02 to 0.05 kWh/m³ for screening and flocculation, 0.15 to 0.30 kWh/m³ for anaerobic digestion, and 0.2 to 0.6 kWh/m³ for microalgae cultivation, depending on lighting and pumping requirements. Energy generation from biogas typically corresponds to 0.25 to 0.35 Nm³ of methane per kilogram of COD removed, equivalent to 1.0 to 1.4 kWh per cubic meter of treated effluent. As a result, the process exhibits a net positive energy balance.

The integrated sequence of screening, acid flocculation, anaerobic digestion, and microalgae cultivation enables high water recovery without the need for energy-intensive membrane processes such as reverse osmosis or nanofiltration. Typical water recovery rates range from 70% to 90% of the initial effluent volume, with recovered water exhibiting residual COD values below 500 to 1000 mg/L, suspended solids below 30 to 50 mg/L, and significantly reduced nitrogen and phosphorus concentrations. The recovered water is suitable for industrial reuse applications such as equipment washing, cooling systems, floor cleaning, and auxiliary process uses.

The microalgal biomass produced constitutes a further valuable output of the process. The biomass typically contains, on a dry basis, from 70 to 85% organic matter, from 4 to 8% nitrogen, from 1 to 3% phosphorus (as P₂O₅ equivalent), from 1 to 2% potassium (as K₂O equivalent), and from 40 to 55% organic carbon, with heavy metal concentrations compatible with agricultural use depending on the initial matrix. The biomass may be valorized as a biofertilizer, soil conditioner, fertigation additive, or as a raw material for nutraceutical, pharmaceutical, cosmetic, chemical, or bioenergy applications. This multi-sector valorization reinforces the technical effect of the disclosed technology by transforming dairy effluents into reusable water, renewable energy, recyclable nutrients, and high-value biomass, thereby achieving a technically robust and quantifiable circular economy model.

In an embodiment, the disclosed technology relates to an integrated process for the valorization of effluents generated by the cheese industry, in particular cheese whey and scotta originating from cheese and ricotta production. The process is configured as a sequential treatment and resource recovery chain designed to convert high-organic-load effluents into reusable water, renewable energy and valued biomass, while avoiding the use of energy-intensive membrane separation technologies.

The process comprises, as a first step, a hydraulic screening stage configured to remove coarse organic solids and undesirable materials from the effluent. The screening step is preferably designed to operate at a design flow rate corresponding to 1.2 to 1.5 times the average effluent flow rate, thereby accommodating peak hydraulic loads. In preferred embodiments, the pressure loss across the screen is maintained below 150 mbar. The screening step is effective in removing fragments of curd, protein coagulates, organic fibres, and residues originating from cleaning-in-place operations, thus protecting downstream physicochemical and biological treatment stages.

Following screening, the effluent is subjected to a controlled acidification step. In this step, the pH of the effluent is adjusted to a value ranging from 4.2 to 5.2, preferably from 4.45 to 4.75. This pH range corresponds to the isoelectric point of casein and results in the precipitation of residual casein and the destabilization of proteinaceous colloids. Acidification is preferably carried out using an acid selected from hydrochloric acid, sulfuric acid, or combinations thereof. For operational safety and homogeneous mixing, the acid is preferably dosed in diluted form, typically at a concentration of about 5 to 10% by mass.

In an embodiment, the acid dosage required to reach the target pH is adjusted according to the nature of the effluent. For cheese whey, the acid consumption typically ranges from 0.5 to 3.0 litres of 33% hydrochloric acid per cubic metre of effluent. For scotta, which often exhibits higher mineralization and buffering capacity, the acid consumption typically ranges from 0.8 to 3.5 litres of 33% hydrochloric acid per cubic metre. These values are preferably refined by titration tests while maintaining the target precipitation pH.

After acidification, the process comprises a flocculation step followed by solid-liquid separation. During flocculation, the precipitated casein and destabilized colloids aggregate into separable flocs. Flocculation is preferably carried out for a duration of 15 to 25 minutes, at an operating temperature from 20 to 30 °C, and with a suspended solids concentration in the range of 200 to 2000 mg/L. The resulting flocs are subsequently separated from the liquid phase, producing a clarified effluent with reduced particulate and colloidal load.

The clarified effluent is then subjected to anaerobic digestion. In a preferred embodiment, digestion is carried out under mesophilic conditions at a temperature from 35 to 38 °C. The hydraulic retention time is preferably from 15 to 30 days, and the organic loading rate is maintained from 2 to 6 kg of chemical oxygen demand per cubic metre per day. Under these conditions, dissolved organic matter is converted into biogas, which preferably contains a methane fraction from 55% to 65%.

Following anaerobic digestion, the liquid digestate is directed to a microalgae cultivation stage. In this stage, microalgae are cultivated using the digestate as a nutrient source, thereby promoting biological removal of nitrogen and phosphorus while generating a valued algal biomass. Cultivation is preferably carried out under controlled photoperiods of 12 to 16 hours of light, with light intensities from 100 to 300 µmol photons per square metre per second, at temperatures from 20 to 30 °C. Carbon dioxide, preferably derived at least in part from the biogas produced during anaerobic digestion, is injected in a controlled manner to regulate pH and support algal growth. Suitable microalgae species include, without limitation, species of the genera Chlorella, Scenedesmus, Spirulina and Nannochloropsis.

The integrated process further comprises a water recovery step, wherein the treated effluent, after anaerobic digestion and microalgae cultivation, is recovered as water suitable for industrial reuse. In an embodiment, the recovered water exhibits a chemical oxygen demand below 1000 mg O₂/L, suspended solids below 50 mg/L, and total nitrogen below 30 mg/L. This water may be reused in non-potable industrial applications, including equipment washing, cooling systems, thermal circuits and fertigation, without requiring polishing by reverse osmosis, nanofiltration or ultrafiltration.

The algal biomass produced by the process constitutes a multi-use intermediate product. In an embodiment, the biomass may be valorized as an agricultural fertilizer or biofertilizer, as a soil conditioner, as an ingredient for fertigation systems, as a raw material for nutraceutical, pharmaceutical, cosmetic or chemical industries, or as a complementary energy substrate. In this context, the algal biomass acts as a vector for reintegration of nutrients and carbon, thereby reinforcing the material circularity of the process.

In an embodiment, the algal biomass exhibits, on a dry weight basis, a nitrogen content from 4 to 8%, a phosphorus content from 1 to 3%, and a potassium content from 1 to 2%, allowing partial substitution of synthetic mineral fertilizers in agricultural applications.

The integrated process enables high resource recovery efficiencies. In an embodiment, water recovery reaches from 70 to 90% of the treated effluent volume, resulting in a reduction of fresh water intake exceeding about 60%. More than approximately 70% of the nitrogen and phosphorus initially present in the effluent may be recovered and valorised, without generating concentrated liquid reject streams or saline wastes.

From an energy perspective, the process is configured to exhibit a net positive energy balance. In preferred embodiments, the total energy consumption of the process is below 1.0 kWh per cubic metre of treated effluent, while the energy recovered in the form of biogas exceeds 1.0 kWh per cubic metre. As a result, the process is clearly distinguished from treatment systems based on reverse osmosis and membrane technologies, which typically exhibit a negative energy balance.

Compared to conventional treatment systems relying on membrane separation, the process described herein achieves a reduction in energy consumption exceedingly approximately 50%, eliminates fouling phenomena and the need for chemical membrane cleaning, avoids the generation of concentrated liquid rejects, and exhibits increased robustness to variations in organic load. Furthermore, the process achieves enhanced circularity of both water and nutrients.

Overall, the integrated process converts effluents from the cheese industry into reusable water, renewable energy and valued algal biomass, establishing a closed-loop circular economy system that is technically integrated, energetically efficient and free from membrane-based separation technologies associated with high energy consumption, technological complexity and elevated capital and operating costs.

In comparative trials without the acidification step, anaerobic digestion exhibited unstable methane production and excessive foaming, whereas the conditioned effluent according to the invention showed stable operation.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following dependent claims further set out particular embodiments of the disclosure.

## Claims

1. System for processing an effluent and cultivating a microalga, comprising:
a primary tank configured to receive the effluent;
a screening unit fluidly connected downstream of the primary tank, the screening unit comprising a sieve and a motor-driven cleaning mechanism configured to remove solid matter retained on the sieve;
a flocculation tank arranged downstream of the screening unit with a container for a flocculant agent fluidly connected to the flocculation tank;
a sand filter arranged downstream of the flocculation tank;
an acidification unit arranged downstream of the sand filter to adjust pH to 4.2-5.2 prior to solid-liquid separation;
a solid-liquid separation unit arranged downstream of the acidification unit and configured to remove precipitated proteins and destabilised colloidal material, thereby producing a clarified effluent;
an anaerobic digestion unit fluidly connected downstream of the solid-liquid separation unit and configured to receive the clarified effluent at an organic loading rate from 2 to 6 kg COD per m³ per day and to convert dissolved organic matter into biogas and a liquid digestate; and
a microalga cultivation unit fluidly configured to receive at least a portion of the liquid digestate and/or carbon dioxide derived from the biogas connected to receive at least a portion of the liquid digestate and/or gaseous products of the anaerobic digestion unit, wherein the acidification unit is configured to adjust the effluent to a pH from 4.2 to 5.2 prior to the solid-liquid separation unit.

2. System according to any of the previous claims, wherein the effluent is an effluent generated by the dairy industry, preferably in production of cheese, more preferably in the production of cheese whey and scotta.

3. System according to any of the previous claims, wherein the sieve is made of ferrous or non-ferrous metals, plastic, ceramics, or composite materials.

4. System according to any of the previous claims, wherein the sieve mesh size is 10-1000 µm; preferably 20-500 µm; preferably 50-400 µm.

5. System according to any of the previous claims, wherein the acidification unit comprising an acid dosing device and a pH control unit comprising at least one pH sensor and a feedback control loop, preferably wherein the acidification unit is configured to adjust the effluent to a pH from 4.45 to 4.75 prior to the solid-liquid separation unit, preferably a pH from 4.5 to 4.6.

6. System according to any of the previous claims, wherein the flocculant agent is an acid; preferably a strong inorganic acid, more preferably wherein the flocculant agent is selected from a list consisting of: hydrochloric acid, sulfuric acid, nitric acid; preferably hydrochloric acid.

7. System according to any of the previous claims, wherein the solid-liquid separation unit comprises a gravitational settler and/or a dissolved air flotation unit.

8. System according to any of the previous claims, wherein the anaerobic digestion unit contains anaerobic microorganisms selected from bacteria and/or archaea, preferably wherein the anaerobic microorganisms are selected from Lactobacillus, Clostridium, Bacteroides, Propionibacterium, Bifidobacterium, Enterococcus, Syntrophomonas, Syntrophobacter, Methanosarcina, Methanobacterium, Methanosaeta, Methanococcus, or combinations thereof.

9. System according to any of the previous claims, wherein the anaerobic digestion generates a biogas; preferably methane and/or carbon dioxide.

10. System according to any of the previous claims, wherein the biogas generated in the anaerobic digestion unit is used as an internal energy source to power at least one unit of the system.

11. System according to any of the previous claims, wherein carbon dioxide separated from the biogas is supplied to the microalga cultivation unit as a carbon source.

12. System according to any of the previous claims, wherein the liquid digestate comprises at least organic matter, nutrients, assimilable nitrogen, phosphorus and water suitable for microalga cultivation.

13. System according to any of the previous claims, wherein the microalga is selected from a list consisting of: *Chlorella, Spirulina, Dunaliella, Scenedesmus, Nannochloropsis, Tetraselmis, Isochrysis, Phaeodactylum, Chlamydomonas, Arthrospiro, Navicula, Botryococcus, Pseudokirchneriella, Cyclotella,* or *Crypthecodinium,* among others.

14. System according to any of the previous claims, wherein the solid-liquid separation unit comprises a flocculation chamber followed by a gravitational settler or a dissolved air flotation unit.

15. Method for processing an effluent and produce microalgae, comprising the following steps:
providing an effluent;
introducing the effluent into a primary tank;
screening the effluent using a sieve to separate a solid fraction from a liquid fraction;
removing retained solids from the sieve using a motor-driven cleaning mechanism;
subjecting the liquid fraction to flocculation by adding a chemical flocculant;
filtering the flocculated liquid fraction using a sand filter;
acidifying the filtered liquid fraction to a pH from 4.2 to 5.2, preferably 4.45 to 4.75 prior to solid-liquid separation;
solid-liquid separating unit configured to remove precipitated proteins and destabilised colloids to produce a clarified effluent;
anaerobic digestion unit operated at 2-6 kg COD·m⁻³·day⁻¹ producing biogas and liquid digestate; and
microalga cultivation unit receiving at least a portion of digestate and/or CO₂ derived from biogas.
